Europäisches Patentamt

European Patent Office

Office européen des brevets

(11) Veröffentlichungsnummer: **0 341 548**

A2

(12) **EUROPÄISCHE PATENTANMELDUNG**

(21) Anmeldenummer: 89107909.7

(22) Anmeldetag: 02.05.89

(51) Int. Cl.⁴: **C07C 69/675** , **C07C 67/31** , **C07C 67/26**

(30) Priorität: 09.05.88 DE 3815826

(43) Veröffentlichungstag der Anmeldung:
15.11.89 Patentblatt 89/46

(84) Benannte Vertragsstaaten:
AT BE CH DE ES FR GB IT LI NL SE

(71) Anmelder: **Henkel Kommanditgesellschaft auf Aktien**
**Postfach 1100 Henkelstrasse 67**
**D-4000 Düsseldorf 1(DE)**

(72) Erfinder: **Stoll, Gerhard, Dr.**
**Danziger Strasse 69**
**D-4052 Korschenbroich 1(DE)**

(54) Verfahren zur Herstellung von vicinal diacyloxysubstituierten Verbindungen.

(57) Vicinal diacyloxysubstituierte Verbindungen der Formeln Ia, Ib, Ic bzw. Id

| | |
|---|---|
| $R^1[vic. (O-CO-R^2)_2]-H$ | (Ia) |
| $R^3[vic. (O-CO-R^2)_2]-CO-OR^4$ | (Ib) |
| $R^3[vic. (O-CO-R^2)_2]-CO-OR^5[vic. (O-CO-R^2)_2]$ | (Ic) |
| $R^6-CO-OR^5[vic. (O-CO-R^2)_2]$ | (Id) |

in denen
$R^1$ einen dreibindigen, gesättigten Kohlenwasserstoffrest mit 6 bis 22 Kohlenstoffatomen,
$R^2$ einen Alkylrest mit 1 bis 3 Kohlenstoffatomen,
$R^3$ einen dreibindigen, gesättigten Kohlenwasserstoffrest mit 10 bis 21 Kohlenstoffatomen,
$R^4$ einen Alkylrest mit 1 bis 22 Kohlenstoffatomen,
$R^5$ einen dreibindigen, gesättigten, Kohlenwasserstoffrest mit 16 bis 22 Kohlenstoffatomen und
$R^6$ einen Alkylrest mit 1 bis 21 Kohlenstoffatomen bedeuten.
lassen sich in einfacher Weise aus den entsprechenden epoxidierten Verbindungen durch Umsetzung mit $C_2$-$C_4$-Carbonsäureanhydriden in Gegenwart von katalytischen Mengen der den Anhydriden entsprechenden Carbonsäuren und/oder katalytischen Mengen Schwefelsäure bei erhöhten Temperaturen herstellen.

EP 0 341 548 A2

## Verfahren zur Herstellung von vicinal diacyloxysubstituierten Verbindungen

Die Erfindung betrifft ein Verfahren zur Herstellung von vicinal diacyloxysubstituierten Verbindungen aus der von Diacyloxyalkanen sowie Estern von Diacyloxyfettsäuren mit Alkanolen und Diacyloxyfettalkoholen und von Estern von Fettsäuren mit Diacyloxyfettalkoholen gebildeten Gruppe der Formeln Ia, Ib, Ic bzw. Id.

| | |
|---|---|
| $R^1$[vic. $(O-CO-R^2)_2$]-H | (Ia) |
| $R^3$[vic. $(O-CO-R^2)_2$]-$CO-OR^4$ | (Ib) |
| $R^3$[vic. $(O-CO-R^2)_2$]-$CO-OR^5$[vic. $(O-CO-R^2)_2$] | (Ic) |
| $R^6$-$CO-OR^5$[vic. $(O-CO-R^2)_2$] | (Id) |

in denen

$R^1$ einen dreibindigen, gesättigten Kohlenwasserstoffrest mit 6 bis 22 Kohlenstoffatomen,

$R^2$ einen Alkylrest mit 1 bis 3 Kohlenstoffatomen,

$R^3$ einen dreibindigen, gesättigten Kohlenwasserstoffrest mit 10 bis 21 Kohlenstoffatomen,

$R^4$ einen Alkylrest mit 1 bis 22 Kohlenstoffatomen,

$R^5$ einen dreibindigen, gesättigten Kohlenwasserstoffrest mit 16 bis 22 Kohlenstoffatomen und

$R^6$ einen Alkylrest mit 1 bis 21 Kohlenstoffatomen bedeuten.

Vicinal diacyloxysubstituierte Verbindungen gehören zum Stand der Technik. Vicinale Di-acyloxyfettsäureester sind unter anderem als Weichmacher (J. Chem. Eng. Data 5, 231 1960)), als synthetische Schmiermittel (Ind. Eng. Chem. 46, 2205 (1954)) sowie in schaumregulierten Wasch-, Reinigungs-und Geschirrspülmitteln (DE-C 24 27 125) eingesetzt worden.

Es sind verschiedene Verfahren zur Herstellung derartiger Di-acyloxyfettsäureester bekannt. So lassen sich z.B. vicinale Diole mit den Anhydriden von Carbonsäuren und/oder mit Carbonsäurehalogeniden acylieren, vgl. die obengenannte Literaturstelle aus Ind. Eng. Chem. 46. Auch die Folgeprodukte der Ringöffnung von Epoxiden mit Carbonsäuren, d.h. die (2-Hydroxyalkyl)ester, lassen sich mit Anhydriden und/oder Säurehalogeniden acylieren, vgl. die obengenannte DE-C 24 27 125 sowie J. Chem. Soc. Perkin Trans. I, 1975, 231 bis 241. Ferner können vicinale Diacylverbindungen aus den entsprechenden Olefinen durch oxidative Acetylierung mittels Pb(IV)acetat, Tl(III)acetat, Hg(II)acetat, Fe(II)persulfat/Essigsäure und Pd(II)-acetat direkt hergestellt werden. In Liebigs Ann. Chem. 138, 297 (1866) wird über die Herstellung von vicinalen Diacetaten durch Reaktion von Epichlorhydrin mit Acetanhydrid im geschlossenen System bei 180°C berichtet.

Es wurde nun gefunden, daß sich vicinal diacyloxysubstituierte Verbindungen der vorstehend genannten Art in besonders einfacher Weise herstellen lassen, indem man Epoxidverbindungen der allgemeinen Formeln IIa, IIb, IIc bzw. IId

| | |
|---|---|
| $R^7$(EpO)-H | (IIa) |
| $R^8$(EpO)-$CO-OR^4$ | (IIb) |
| $R^8$(EpO)-$CO-OR^9$(EpO) | (IIc) |
| $R^6$-$CO-OR^9$(EpO) | (IId) |

in denen

$R^7$(EpO) einen epoxidierten Alkenylrest mit 6 bis 22 Kohlenstoffatomen,

$R^8$ (EpO) einen epoxidierten Alkenylrest mit 10 bis 21 Kohlenstoffatomen und

$R^9$(EpO) einen epoxidierten Alkenylrest mit 16 bis 22 Kohlenstoffatomen

bedeuten sowie $R^4$ und $R^6$ wie oben definiert sind,

mit $C_2$-$C_4$-Carbonsäureanhydriden der Formel III

$(R^2-CO-)_2O$ (III)

in der

$R^2$ wie oben definiert ist,

in Gegenwart von katalytischen Mengen der den Carbonsäureanhydriden entsprechenden Carbonsäuren der Formel $R^2$-COOH und/oder katalytischen Mengen Schwefelsäure bei erhöhten Temperaturen umsetzt.

Die als Ausgangsverbindungen für das Verfahren der Erfindung eingesetzten Epoxidverbindungen der allgemeinen Formeln IIa, IIb, IIc bzw. IId lassen sich durch übliche Epoxidation von ungesättigten Verbin-

2

dungen der allgemeinen Formel IVa, IVb, IVc bzw. IVd

| $R^7$-H | (IVa) |
|---|---|
| $R^8$-CO-O$R^4$ | (IVb) |
| $R^8$-CO-O$R^9$ | (IVc) |
| $R^6$-CO-O$R^9$ | (IVd) |

herstellen, in denen $R^4$ und $R^6$ wie oben definiert sind und

$R^7$ einen Alkenylrest mit 6 bis 22 Kohlenstoffatomen,
$R^8$ einen Alkenylrest mit 10 bis 21 Kohlenstoffatomen und
$R^9$ einen Alkenylrest mit 16 bis 22 Kohlenstoffatomen
bedeuten.

Die Alkene der Formel IVa, die geradkettig oder verzweigt sein können, sind bekannte Verbindungen; typische Vertreter sind Hexen, Octen, Decen, Undecen, Dodecen, Tetradecen, Hexadecen, Octadecen, Eicosen und Docosen.

Als Ausgangsverbindungen der Formel IVb werden Ester von ungesättigten, geradkettigen oder verzweigten, insbesondere geradkettigen Fettsäuren mit Monoalkanolen mit 1 bis 22 Kohlenstoffatomen, beispielsweise mit Methanol, Ethanol, n-Propanol, n-Butanol, n-Pentanol, n-Hexanol, n-Octanol, n-Decanol, n-Dodecanol, n-Tetradecanol, n-Hexadecanol, n-Octadecanol, n-Eicosanol und n-Docosanol sowie deren verzweigte Isomere wie z.B. 3-Methylbutanol (Isoamylalkohol) und 2-Ethylhexanol (Isooctanol), eingesetzt. Ungesättigte Fettsäuren der Formel $R^8$-COOH können synthetischen, z.B. Undecylensäure, oder natürlichen Ursprungs, z.B. Palmitolein-, Öl-, Petroselin-, Gadolein- und/oder Erucasäure, sein. Derartige ungesättigte Fettsäuren sind insbesondere aus natürlichen Fetten bzw. Ölen zugänglich, z.B. aus Sonnenblumen-, Soya-, Raps- und Korianderöl; weiterhin auch aus tierischen Fetten wie Rindertalg und Schweineschmalz. Sie werden im allgemeinen in Form ihrer technischen Gemische zu den Fettsäureestern der Formel IVb verarbeitet; demgemäß werden bevorzugt für das Verfahren der Erfindung technische Gemische der epoxidierten Fettsäureester der Formel IVb eingesetzt. Die Monoalkanolkomponente der Fettsäureester der allgemeinen Formel IVb kann von Methanol, Ethanol, Propanol, und Butanol einschließlich der Isomeren derselben abgeleitet sein.

Die Ausgangsverbindungen der allgemeinen Formel IVc leiten sich ab von den Fettsäuren $R^8$-COOH mit den vorstehend genannten Bedeutungen und ungesättigten geradkettigen oder verzweigten, insbesondere geradkettigen Fettalkoholen synthetischen und insbesondere natürlichen Ursprungs; typische Beispiele für diese Fettalkohole sind Palmitoleyl-, Oleyl-, Elaidinyl-, Gadoleyl- und Erucylalkohol, einschließlich der in der Fettchemie üblichen technischen Gemische derselben.

Die Ausgangsverbindungen der allgemeinen Formel IVd sind Ester von gesättigten, geradkettigen oder verzweigten, insbesondere geradkettigen Fettsäuren mit 1 bis 22 Kohlenstoffatomen mit den oben erläuterten ungesättigten Fettalkoholen $R^9$-OH. Typische Vertreter für die Fettsäuren der Formel $R^6$-CO-OH sind Essig-, Propion-, Butter-, Valerian-, Capron-, Önath-, Capryl-, Pelargon-, Caprin-, Hendecan-, Laurin-, Myristin-, Palmitin-, Stearin-, Araduin und Behensäure. Für die höheren Carbonsäuren aus dieser, Reihe ($C_6$ bis $C_{22}$) können die in der Fettchemie üblichen technischen Gemische dieser Fettsäuren synthetischen und insbesondere natürlichen Ursprungs eingesetzt werden.

Je nach dem Aufbau der ungesättigten Verbindungen der allgemeinen Formel IVb, IVc und IVd erhält man bei der Epoxidation Verbindungen, die nur im Fettsäureteil, nur im Fettalkoholteil oder sowohl im Fettsäureteil als auch im Fettalkoholteil epoxidiert sind. Technische Gemische der ungesättigten Verbindungen können, insbesondere wenn sie, wie in der Fettchemie üblich, aus natürlichen Rohstoffen hergestellt werden, Fettsäureester enthalten, die im Fettsäure- und/oder Fettalkoholteil mehr als eine olefinische Doppelbindung aufweisen. Typische Beispiele sind die entsprechenden Derivate von Linol- und Linolensäure bzw. Linoleylalkohol und Linolenylalkohol, die jeweils z.B. aus Leinöl zugänglich sind. Bei derartigen Ausgangsverbindungen können nach der Epoxidation und anschließender Ringöffnung gemäß dem Verfahren der Erfindung Gemische entstehen, die neben den Verbindungen der Formeln Ib bis Id auch gewisse Anteile an Verbindungen enthalten, die Ringöffnungsprodukte von Epoxidverbindungen mit mehr als einer bzw. mehr als zwei Epoxidfunktionen darstellen. Entsprechendes gilt, wenn man von technischen Gemischen der Alkene IVa ausgeht.

Ein besonders günstiges Verfahren für die Herstellung der als Ausgangsverbindungen im Verfahren der Erfindung verwendeten Epoxidverbindungen ist die Epoxidation mit "in situ"-gebildeter Perameisensäure, z.B. gemäß DE-A 30 02 861 und US-C 2 485 160.

In dem Verfahren der Erfindung werden die Epoxidverbindungen der allgemeinen Formel IIa bis IId mit

3

Anhydriden von Carbonsäuren mit 2 bis 4 Kohlenstoffatomen, z.B. Propion-oder Buttersäureanhydrid, besonders bevorzugt jedoch mit Essigsäureanhydrid, umgesetzt, wobei die Reaktion in Gegenwart von wirksamen Mengen der den eingesetzten Anhydriden entsprechenden Carbonsäuren und/oder Schwefelsäure bei erhöhten Temperaturen unter Atmosphärendruck durchgeführt wird. Bevorzugte Mengen für die eingesetzten Carbonsäuren sind 0,5 bis 15 Mol-%. Anstelle von bzw. zusammen mit den vorgenannten Carbonsäuren können auch katalytische Mengen Schwefelsäure, insbesondere 0,1 bis 3 Mol-%, zugesetzt werden. Die angegebenen Molprozente beziehen sich auf die Mole der in den Ausgangsverbindungen vorhandenen Epoxidfunktionen.

Gemäß einer vorteilhaften Ausführungsform des Verfahrens der Erfindung führt man die Umsetzung unter Umgebungsdruck am Siedepunkt des jeweiligen Reaktionssystems durch. Weiterhin ist bevorzugt, daß die Reaktion lösemittelfrei durchgeführt wird, wobei allenfalls das eingesetzte Carbonsäureanhydrid als Lösemittel im Überschuß eingesetzt wird. Nach der Umsetzung wird das Reaktionssystem mit wasserfreien Basen neutralisiert, z.B. mit Natriumalkoholat. Überschüssiges bzw. nicht umgesetztes $C_2$-$C_4$-Carbonsäureanhydrid wird bevorzugt durch Destillation, gegebenenfalls unter vermindertem Druck entfernt. Die bei der Neutralisation der als Katalysatoren eingesetzten Carbonsäuren und/oder Schwefelsäure gebildeten Neutralisationsprodukte fallen aus und können in einfacher Weise durch Filtration entfernt werden.

Das Verfahren der Erfindung wird im folgenden anhand bevorzugter Ausführungsbeispiele näher erläutert. Die angegebenen Mengen für Carbonsäuren und/oder Schwefelsäure in Mol-% beziehen sich auf Mole vorhandener Epoxidfunktionen.

Beispiel 1.

vic. Diacetyl-stearinsäuremethylester

6 Mol eines epoxidierten Ölsäuremethylesters (Epoxid-Sauerstoffgehalt: 4,61 %) wurde mit 12 Mol Acetanhydrid, 10 Mol-% Essigsäure und 1 Mol-% konzentrierter Schwefelsäure 4 Stunden lang unter heftigem Rückfluß gerührt. Nach Neutralisation mit Natriummethylat wurde das überschüssige Acetanhydrid abdestilliert; der Rückstand wurde im Wasserstrahlvakuum (ca. 15 Torr) zur Entfernung von flüchtigen Anteilen Acetanhydrids bis 120° C destilliert. Ausgefallene Salze wurden durch Filtration von dem Destillationsrückstand entfernt; man erhielt eine braune klare Flüssigkeit mit den folgenden analytischen Daten:

| Epoxid-O-Gehalt: | 0,32 % |
|---|---|
| OHZ: | 2,6 |
| VZ: | 294,4 |
| SZ: | 1,1 |
| IZ: | 14,7 |

Beispiel 2.

vic. Diacetyl-stearinsäure-i-octylester

1 Mol eines epoxidierten Ölsäureisooctylesters (Epoxid-Sauerstoffgehalt: 3,73 %) wurde mit 2 Mol Essigsäureanhydrid, 10 Mol-% Essigsäure und 1 Mol-% konzentrierter Schwefelsäure 6 h unter Rückfluß gerührt. Nach Neutralisation mit Natriummethylat wurde das überschüssige Essigsäureanhydrid abdestilliert; der Rückstand wurde bis 120° C im Ölpumpenvakuum zur Entfernung flüchtiger Anteile destilliert. Der Destillationsrückstand wurde durch Filtration von ausgefallenen Salzen befreit; man erhielt eine gelb-braune, klare Flüssigkeit mit den folgenden analytischen Daten:

4

| Epoxid-O-Gehalt: | 0,36 % |
|---|---|
| OHZ: | 1,1 |
| VZ: | 232,7 (theor.: 325) |
| SZ: | 0,6 |
| IZ: | .10,0 |

Die erhaltene Verbindung zeigte eine ausgeprägte schauminhibierende Wirkung und war zudem biologisch sehr gut abbaubar.

Beispiel 3.

vic. Dipropionyl-stearinsäure-isooctylester

In der in Beispiel 2 beschriebenen Weise wurden
1 Mol epoxidierter Ölsäureisooctylester (Epoxid-O-Gehalt 3,82 %),
2 Mol Propionsäureanhydrid,
10 Mol-% Propionsäure und
1 Mol-% konzentrierte Schwefelsäure

6 h bei 150° C umgesetzt. Nach Entfernung flüchtiger Anteile und Filtration erhielt man ein Produkt mit den folgenden analytischen Daten:

| Epoxid-O-Gehalt: | 0,20 % |
|---|---|
| OHZ: | 0,6 |
| SZ: | 1,2 |
| VZ: | 240,8 |
| IZ: | 8,6 |

Beispiel 4.

vic. Dibutanoyl-stearinsäure-isooctylester

In der in Beispiel 2 beschriebenen Weise wurden
1 Mol epoxidierter Ölsäureisooctylester (Epoxid-O-Gehalt 3,82 %),
2 Mol Buttersäureanhydrid,
10 Mol-% Buttersäure und
1,5 Mol-% konzentrierte Schwefelsäure

6,5 h bei 150° C umgesetzt. Nach dem Abdestillieren flüchtiger Bestandteile bis 135° C im Ölpumpenvakuum (weniger als 1 Torr) erhielt man ein Produkt mit den folgenden analytischen Daten:

| Epoxid-O-Gehalt: | 0,20 % |
|---|---|
| OHZ: | 2,6 |
| SZ: | 1,5 |
| VZ: | 237,6 |
| IZ: | 8,8 |

Beispiel 5.

vic. Diacetyl-sojafettsäure-isooctylester

6 Mol epoxidierter Sojafettsäureisooctylester (Epoxid-O-Gehalt 4,99 %),
12 Mol Acetanhydrid,
10 Mol-% Essigsäure und
1 Mol-% konzentrierte Schwefelsäure
wurden in der in Beispiel 1 beschriebenen Weise 7,5 h unter starkem Rückflußkochen umgesetzt. Das Reaktionsprodukt wurde im Ölpumpenvakuum (weniger als 1 Torr) bis 125°C destilliert. Der dunkelbraune Rückstand, der mit Wasserstoffperoxid gebleicht werden konnte, wies nach der Abfiltration von ausgefallenen Salzen die folgenden analytischen Daten auf:

| Epoxid-O-Gehalt: | 0,23 % |
|---|---|
| VZ: | 252,1 |
| SZ: | 1,2 |
| OHZ: | 2,8 |
| IZ: | 20,4 |

Beispiel 6.

vic. Dipropionyl-stearinsäureisooctylester

1 Mol eines epoxidierten Ölsäureisooctylesters (Epoxid-Sauerstoffgehalt: 3,28 %) wurde mit 2 Mol Propionsäureanhydrid und 2,0 g konzentrierter Schwefelsäure (2 Mol-%, bezogen auf Epoxidgehalt) 8 h unter Rückfluß (ca. 150°C) gerührt; das Reaktionsgemisch wurde mit 7,4 g Natriummethylat neutralisiert. Der Überschuß des Propionsäureanhydrids wurde im Vakuum bei 15 Torr abdestilliert; der Destillationsrückstand wurde im Vakuum bis 120°C zur Entfernung flüchtiger Anteile destilliert. Der Destillationsrückstand wurde durch Filtration von ausgefallenen Salzen befreit; man erhielt eine klare, dunkelbraune Flüssigkeit mit den folgenden analytischen Daten:

| Epoxid-O-Gehalt: | 0,30 % |
|---|---|
| OHZ: | 0,4 |
| SZ: | 2,1 |
| VZ: | 235,3 |
| IZ: | 12,4 |

## Ansprüche

1. Verfahren zur Herstellung von vicinal diacyloxysubstituierten Verbindungen aus der von Diacyloxyalkanen sowie Estern von Diacyloxyfettsäuren mit Alkanolen und Diacyloxyfettalkoholen und von Estern von Fettsäuren mit Diacyloxyfettalkoholen gebildeten Gruppe der Formeln Ia, Ib, Ic bzw. Id.

| $R^1[vic. (O\text{-}CO\text{-}R^2)_2]\text{-}H$ | (Ia) |
|---|---|
| $R^3[vic. (O\text{-}CO\text{-}R^2)_2]\text{-}CO\text{-}OR^4$ | (Ib) |
| $R^3[vic. (O\text{-}CO\text{-}R^2)_2]\text{-}CO\text{-}OR^5[vic. (O\text{-}CO\text{-}R^2)_2]$ | (Ic) |
| $R^6\text{-}CO\text{-}OR^5[vic. (O\text{-}CO\text{-}R^2)_2]$ | (Id) |

in denen
$R^1$ einen dreibindigen, gesättigten Kohlenwasserstoffrest mit 6 bis 22 Kohlenstoffatomen,
$R^2$ einen Alkylrest mit 1 bis 3 Kohlenstoffatomen,
$R^3$ einen dreibindigen, gesättigten Kohlenwasserstoffrest mit 10 bis 21 Kohlenstoffatomen,

R⁴ einen Alkylrest mit 1 bis 22 Kohlenstoffatomen,

R⁵ einen dreibindigen, gesättigten Kohlenwasserstoffrest mit 16 bis 22 Kohlenstoffatomen und

R⁶ einen Alkylrest mit 1 bis 21 Kohlenstoffatomen

bedeuten,

dadurch gekennzeichnet, daß man Epoxidverbindungen der allgemeinen Formeln IIa, IIb, IIc bzw. IId

| | |
|---|---|
| R⁷(EpO)-H | (IIa) |
| R⁸(EpO)-CO-OR⁴ | (IIb) |
| R⁸(EpO)-CO-OR⁹(EpO) | (IIc) |
| R⁶-CO-OR⁹(EpO) | (IId) |

in denen

R⁷(EpO) einen epoxidierten Alkenylrest mit 6 bis 22 Kohlenstoffatomen,

R⁸(EpO) einen epoxidierten Alkenylrest mit 10 bis 21 Kohlenstoffatomen und

R⁹(EpO) einen epoxidierten Alkenylrest mit 16 bis 22 Kohlenstoffatomen

bedeuten sowie R⁴ und R⁶ wie oben definiert sind, mit C₂-C₄-Carbonsäureanhydriden der Formel III

(R²-CO-)₂O      (III)

in der

R² wie oben definiert ist,

in Gegenwart von katalytischen Mengen der den Carbonsäureanhydriden entsprechenden Carbonsäuren der Formel R²-COOH und/oder katalytischen Mengen Schwefelsäure bei erhöhten Temperaturen umsetzt.

2. Verfahren nach Anspruch 1, dadurch gekennzeichnet, daß man die Umsetzung in Gegenwart von 0,5 bis 15 Mol-% der den Carbonsäureanhydriden entsprechenden Carbonsäuren und/oder 0,1 bis 3 Mol-% Schwefelsäure pro Mol vorhandener Epoxidfunktionen durchführt.

3. Verfahren nach Anspruch 1 oder 2, dadurch gekennzeichnet, daß man die Umsetzung unter Umgebungsdruck am Siedepunkt des Reaktionssystems durchführt.

4. Verfahren nach mindestens einem der Ansprüche 1 bis 3, dadurch gekennzeichnet, daß man nach der Umsetzung das Reaktionssystem mit wasserfreien Basen neutralisiert, überschüssiges bzw. nicht umgesetztes C₂-C₄-Carbonsäureanhydrid durch Destillation, gegebenenfalls unter vermindertem Druck, entfernt und die Neutralisationsprodukte abfiltriert.